# EUROPEAN PATENT SPECIFICATION

(11) **EP 2 757 970 B1**
(45) Date of publication and mention of the grant of the patent: **19.08.2020**
(21) Application number: 12766558.6
(22) Date of filing: 19.09.2012
(51) Int. Cl.: A61B 17/072, A61B 17/00, A61B 17/29, A61B 17/32

(54) **ADJUNCT THERAPY DEVICE FOR APPLYING HEMOSTATIC AGENT**
ADJUVANTE THERAPIEVORRICHTUNG ZUR ANWENDUNG EINES HÄMOSTATISCHEN MITTELS
DISPOSITIF DE THÉRAPIE COMPLÉMENTAIRE POUR APPLIQUER UN AGENT HÉMOSTATIQUE

(30) Priority: 22.09.2011 US 201113240141
(43) Date of publication of application: 30.07.2014
(73) Proprietor: Ethicon Endo-Surgery, Inc., Cincinnati, OH 45242 (US)
(72) Inventor: WEISENBURGH, William B., II, Maineville, Ohio 45039 (US); SMITH, Craig S., Cincinnati, Ohio 45208 (US); BLAIR, Gregory B., San Jose, California 95125 (US); HUANG, Zhifan F., Mason, Ohio 45040 (US); HOFFMAN, Douglas B., Harrison, Ohio 45030 (US); GEIER, Kristi S., Morrow, Ohio 45152 (US); SMITH, Bret W., Kings Mills, Ohio 45034 (US); LYTLE, Thomas W., IV, Liberty Township, Ohio 45044 (US); OVERMYER, Mark D., Cincinnati, Ohio 45236 (US); BEAR, Brian W., Cincinnati, Ohio 45241 (US); SETSER, Michael E., Burlington, Kentucky 41005 (US); LE, Thu Anh, Bridge Water, New Jersey 08807 (US); WOODARD, James A., Jr., Mason, Ohio 45040 (US); MODI, Kreena R., Akron, Ohio 44321 (US); ZAVATSKY, Joseph, Flemington, New Jersey 08822 (US)
(74) Representative: Tunstall, Christopher Stephen
(86) International application number: PCT/US2012/056057
(87) International publication number: WO 2013/043687

(56) References cited:
- EP-A1- 2 090 231
- US-A1- 2005 070 929
- US-A1- 2005 192 628
- US-A1- 2006 111 738
- US-A1- 2006 271 104
- US-A1- 2008 110 961
- US-A1- 2009 120 994
- US-A1- 2011 114 701
- US-B1- 6 325 810
- US-B2- 7 721 930

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in; U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,964,363, entitled "Surgical Stapling Instrument having Articulation Joint Support Plates for Supporting a Firing Bar," issued November 15, 2005; U.S. Pat. No. 6,978,921, entitled "Surgical Stapling Instrument Incorporating an E-Beam Firing Mechanism," issued December 27, 2005; U.S. Pat. No. 6,988,649, entitled "Surgical Stapling Instrument Having a Spent Cartridge Lockout," issued January 24, 2006; U.S. Pat. No. 7,000,818, entitled "Surgical Stapling Instrument Having Separate Distinct Closing and Firing Systems," issued February 21, 2006; U.S. Pat. No. 7,111,769, entitled "Surgical Instrument Incorporating an Articulation Mechanism having Rotation about the Longitudinal Axis," issued September 26, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument having Multistroke Firing with Opening Lockout," issued October 14, 2008; U.S. Pat. No. 7,721,930, entitled "Disposable Cartridge with Adhesive for Use with a Stapling Device," issued May 25, 2010; and U.S. Pat. No. 7,455,208, entitled "Surgical Instrument with Articulating Shaft with Rigid Firing Bar Supports," issued November 25, 2008.

While the surgical staplers referred to above are described as being used in endoscopic procedures, it should be understood that such surgical staplers may also be used in open procedures and/or other non-endoscopic procedures.

EP 2 090 231 A1 discloses a surgical stapler with a buttress material configured to be releasably attached to a staple cartridge.
US2011/0114701 A1 discloses a surgical stapler with a wound treatment material delivery pouch in the anvil.

While various kinds of surgical stapling instruments and associated components have been made and used, it is believed that no one prior to the inventor(s) has made or used the invention described in the appended claims.

### BRIEF DESCRIPTION OF THE DRAWINGS

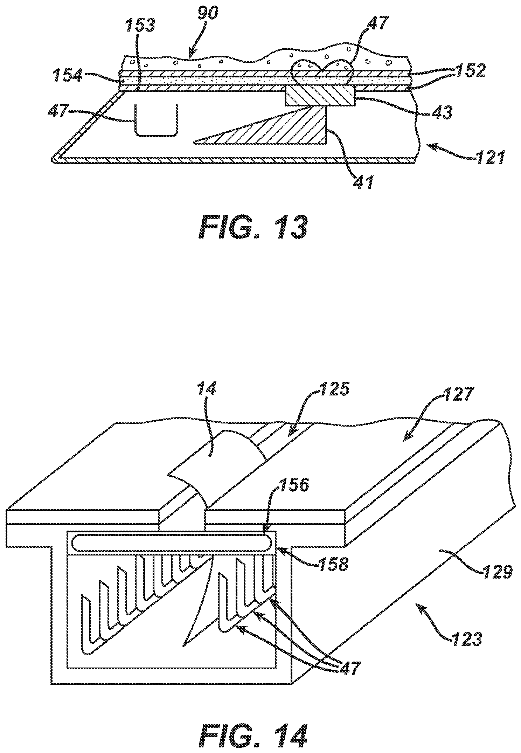

The accompanying drawings, which are incorporated in and constitute a part of this specification, illustrate embodiments of the disclosure and, together with the general description given above, and the detailed description of the embodiments given below, serve to explain the principles of the present invention. In particular Fig. 13 and 14 disclose an embodiment of the invention.
FIG. 1A depicts a perspective view of an articulating surgical instrument with an end effector in a nonarticulated position;
FIG. 1B depicts a perspective view of the surgical instrument of FIG. 1A with an end effector in an articulated position;
FIG. 2 depicts a perspective view of an opened end effector of the surgical instrument of FIGS. 1A-1B;
FIG. 3A depicts a side cross-sectional view of the end effector of FIG. 2, taken along line 3-3 of FIG. 2, with the firing bar in a proximal position;
FIG. 3B depicts a side cross-sectional view of the end effector of FIG. 2, taken along line 3-3 of FIG. 2, but showing the firing bar in a distal position;
FIG. 4 depicts an end cross-sectional view of the end effector of FIG. 2, taken along line 4-4 of FIG. 2;
FIG. 5 depicts an exploded perspective view of the end effector of FIG. 2;
FIG. 6 depicts a perspective view of the end effector of FIG. 2, positioned at tissue and having been actuated once in the tissue;
FIG. 7 depicts a perspective view of a version of removable cartridge of the end effector of FIG. 2 with an exemplary buttress disposed above the cartridge;
FIG. 8 depicts a detail view of an exemplary staple from the end effector of FIG. 2 being actuated through tissue;
FIG. 9 depicts a perspective view of an alternative version of an anvil of an upper jaw and cartridge in a lower jaw of the end effector of FIG. 2 and an exemplary applicator to dispense a biocompatible material onto the anvil and lower jaw;
FIG. 10 depicts an elevation view of the end effector of FIG. 9 with the biocompatible material dispensed onto the anvil and lower jaw;
FIG. 11A depicts a cross-sectional end view of the dispenser of FIG. 9 taken along line 11-11 of FIG. 9;
FIG. 11B depicts a cross-sectional end view of an alternate version of the dispenser of FIG. 9 taken along line 11-11 of FIG. 9;
FIG. 12 depicts an elevation view of an exemplary staple released from the end effector of FIG. 10 into tissue;
FIG. 13 depicts a cross-sectional side view of an exemplary removable cartridge inserted into a lower jaw of the end effector of FIG. 2 inserted a staple through an exemplary buttress and into tissue;
FIG. 14 depicts a fragmentary, perspective view of the cartridge of FIG. 13;
FIG. 15 depicts a perspective view of an end effector with an anvil cartridge including a tissue repair composition, the end effector positioned at and actuated within the tissue to release the tissue repair composition onto the tissue.

The drawings are not intended to be limiting in any way, and it is contemplated that various embodiments may be carried out in a variety of other ways, including those not necessarily depicted in the drawings. The accompanying drawings incorporated in and forming a part of the specification illustrate several aspects of the present disclosure, and together with the description serve to explain the principles of the invention; it being understood, however, that this invention is not limited to the precise arrangements shown but is defined by the wording of the claims.

### DETAILED DESCRIPTION

The following description of certain examples of the disclosure should not be used to limit the scope of the present invention. Other examples, features, aspects, embodiments, and advantages of the invention will become apparent to those skilled in the art from the following description, which is by way of illustration. Accordingly, the drawings and descriptions should be regarded as illustrative in nature and not restrictive.

### I. Exemplary Surgical Stapler

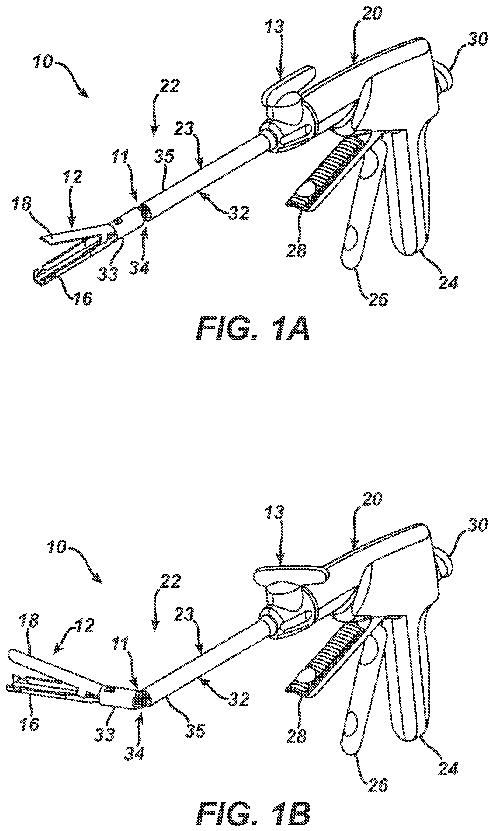

FIGS. 1-6 depict an exemplary surgical stapling and severing instrument (10) that is sized for insertion, in a nonarticulated state as depicted in FIG. 1A, through a trocar cannula passageway to a surgical site in a patient for performing a surgical procedure. Surgical stapling and severing instrument (10) includes handle portion (20) connected to implement portion (22), the latter further comprising shaft (23) distally terminating in an articulation mechanism (11) and a distally attached end effector (12). Once articulation mechanism (11) and end effector (12) are inserted through the cannula passageway of a trocar, articulation mechanism (11) may be remotely articulated, as depicted in FIG. 1B, by articulation control (13). Thereby, end effector (12) may reach behind an organ or approach tissue from a desired angle or for other reasons. It should be understood that terms such as "proximal" and "distal" are used herein with reference to a clinician gripping handle portion (20) of instrument (10). Thus, end effector (12) is distal with respect to the more proximal handle portion (20). It will be further appreciated that for convenience and clarity, spatial terms such as "vertical" and "horizontal" are used herein with respect to the drawings. However, surgical instruments are used in many orientations and positions, and these terms are not intended to be limiting and absolute.

End effector (12) of the present example includes a lower jaw (16) and a pivotable anvil (18). Handle portion (20) includes pistol grip (24) toward which closure trigger (26) is pivotally drawn by the clinician to cause clamping, or closing, of the anvil (18) toward lower jaw (16) of end effector (12). Such closing of anvil (18) is provided through an outmost closure sleeve (32), which longitudinally translates relative to handle portion (20) in response to pivoting of closure trigger (26) relative to pistol grip (24). A distal closure ring (33) of closure sleeve (32) is indirectly supported by frame (34) of implement portion (22). At articulation mechanism (11), a proximal closure tube (35) of closure sleeve (32) communicates with the distal closure ring (33). Frame (34) is flexibly attached to lower jaw (16) via articulation mechanism (11), enabling articulation in a single plane. Frame (34) also longitudinally slidingly supports a firing drive member (not shown) that extends through shaft (23) and communicates a firing motion from firing trigger (28) to firing bar (14). Firing trigger (28) is farther outboard of closure trigger (26) and is pivotally drawn by the clinician to cause the stapling and severing of clamped tissue in end effector (12), as will be described in greater detail below. Thereafter, release button (30) is depressed to release the tissue from end effector (12).

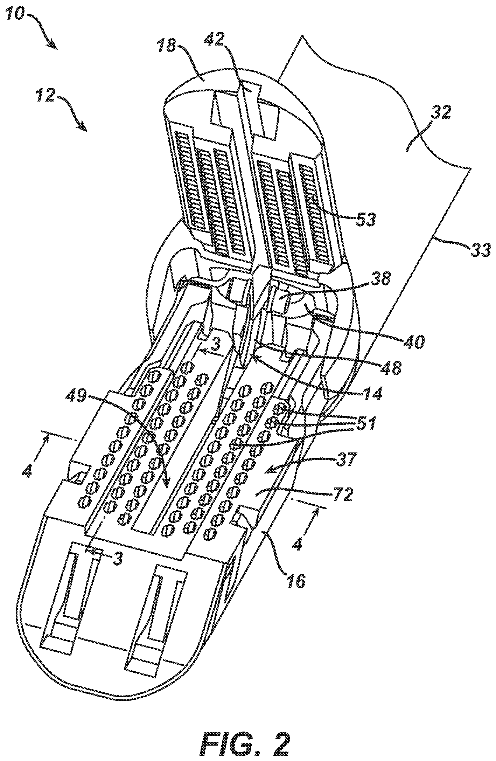

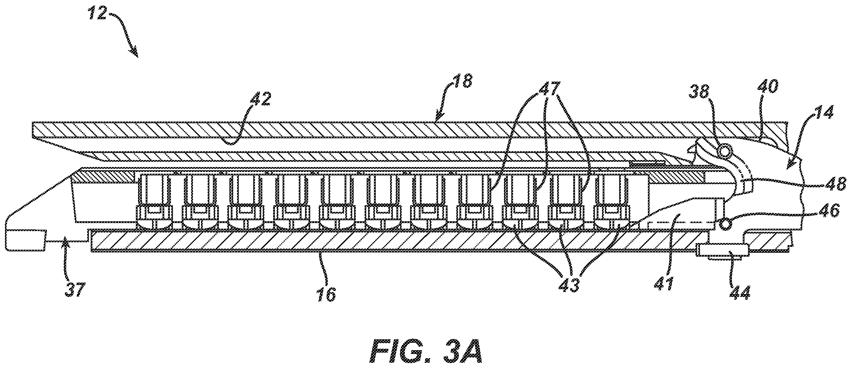

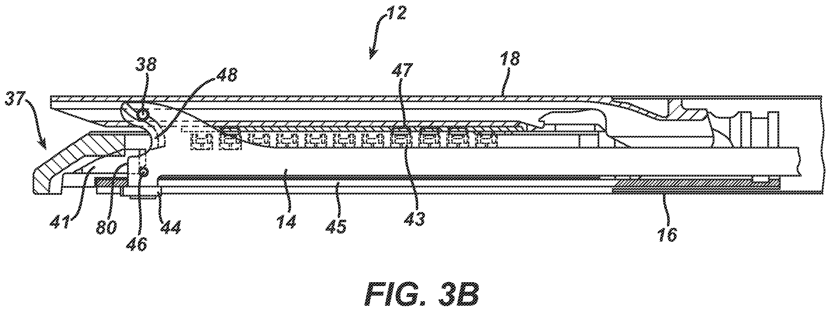

FIGS. 2-5 depict end effector (12) employing an E-beam firing bar (14) to perform a number of functions. As best seen in FIGS. 3A-3B, firing bar (14) includes a transversely oriented upper pin (38), a firing bar cap (44), a transversely oriented middle pin (46), and a distally presented cutting edge (48). Upper pin (38) is positioned and translatable within an anvil pocket (40) of anvil (18). Firing bar cap (44) slidably engages a lower surface of lower jaw (16) by having firing bar (14) extend through channel slot (45) (shown in FIG. 3B) that is formed through lower jaw (16). Middle pin (46) slidingly engages a top surface of lower jaw (16), cooperating with firing bar cap (44). Thereby, firing bar (14) affirmatively spaces end effector (12) during firing, overcoming pinching that may occur between anvil (18) and lower jaw (16) with a minimal amount of clamped tissue and overcoming staple malformation with an excessive amount of clamped tissue.

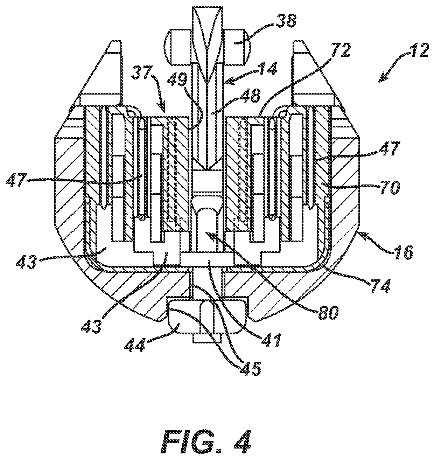

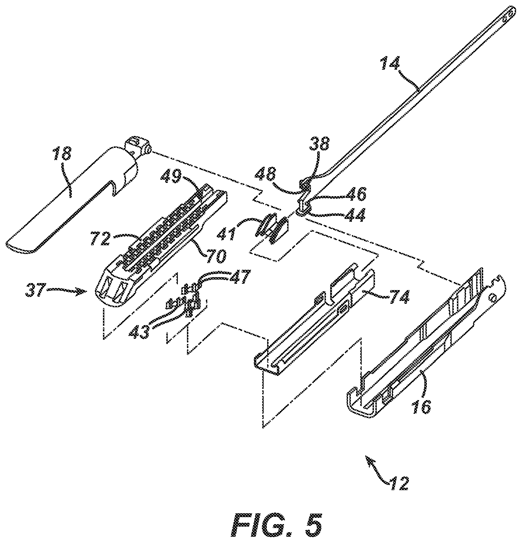

FIG. 2 shows firing bar (14) proximally positioned and anvil (18) pivoted to an open position, allowing an unspent staple cartridge (37) to be removably installed into a channel of lower jaw (16). As best seen in FIGS. 4-5, staple cartridge (37) of this example includes a cartridge body (70), which presents an upper deck (72) and is coupled with a lower cartridge tray (74). As best seen in FIG. 2, a vertical slot (49) is formed through part of staple cartridge (37). As also best seen in FIG. 2, three rows of staple apertures (51) are formed through upper deck (72) on one side of vertical slot (49), with another set of three rows of staple apertures (51) being formed through upper deck (72) on the other side of vertical slot (49). Referring back to FIGS. 3-5, a wedge sled (41) and a plurality of staple drivers (43) are captured between cartridge body (70) and tray (74), with wedge sled (41) being located proximal to staple drivers (43). Wedge sled (41) is movable longitudinally within staple cartridge (37); while staple drivers (43) are movable vertically within staple cartridge (37). Staples (47) are also positioned within cartridge body (70), above corresponding staple drivers (43). In particular, each staple (47) is driven vertically within cartridge body (70) by a staple driver (43) to drive staple (47) out through an associated staple aperture (51). As best seen in FIGS. 3A-3B and 5, wedge sled (41) presents inclined cam surfaces that urge staple drivers (43) upwardly as wedge sled (41) is driven distally through staple cartridge (37).

With end effector (12) closed as depicted in FIG. 3A, firing bar (14) is advanced in engagement with anvil (18) by having upper pin (38) enter a longitudinal anvil slot (42). A pusher block (80) is located at the distal end of firing bar (14), and is configured to engage wedge sled (41) such that wedge sled (41) is pushed distally by pusher block (80) as firing bar (14) is advanced distally through staple cartridge (37). During such firing, cutting edge (48) of firing bar (14) enters vertical slot (49) of staple cartridge (37), severing tissue clamped between staple cartridge (37) and anvil (18). As shown in FIGS. 3A-3B, middle pin (46) and pusher block (80) together actuate staple cartridge (37) by entering into slot (49) within staple cartridge (37), driving wedge sled (41) into upward camming contact with staple drivers (43) that in turn drive staples (47) out through staple apertures (51) and into forming contact with staple forming pockets (53) on the inner surface of anvil (18). FIG. 3B depicts firing bar (14) fully distally translated after completing severing and stapling tissue.

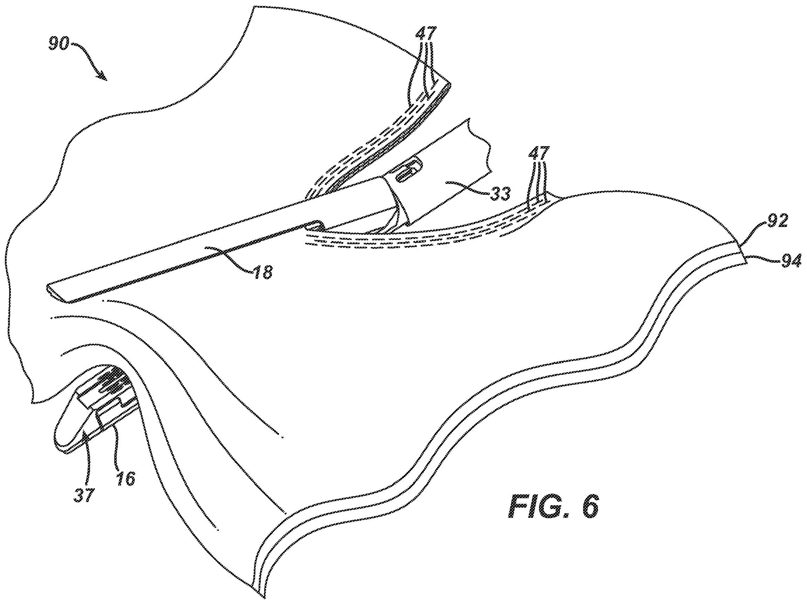

FIG. 6 shows end effector (12) having been actuated through a single stroke through tissue (90). Cutting edge (48) has cut through tissue (90), while staple drivers (43) have driven three alternating rows of staples (47) through the tissue (90) on each side of the cut line produced by cutting edge (48). Staples (47) are all oriented substantially parallel to the cut line in this example, though it should be understood that staples (47) may be positioned at any suitable orientations. In the present example, end effector (12) is withdrawn from the trocar after the first stroke is complete, spent staple cartridge (37) is replaced with a new staple cartridge, and end effector (12) is then again inserted through the trocar to reach the stapling site for further cutting and stapling. This process may be repeated until the desired amount of cuts and staples (47) have been provided. Anvil (18) may need to be closed to facilitate insertion and withdrawal through the trocar; and anvil (18) may need to be opened to facilitate replacement of staple cartridge (37).

It should be understood that cutting edge (48) may sever tissue substantially contemporaneously with staples (47) being driven through tissue during each actuation stroke. In the present example, cutting edge (48) just slightly lags behind driving of staples (47), such that a staple (47) is driven through the tissue just before cutting edge (48) passes through the same region of tissue, though it should be understood that this order may be reversed or that cutting edge (48) may be directly synchronized with adjacent staples. While FIG. 6 shows end effector (12) being actuated in two layers (92, 94) of tissue (90), it should be understood that end effector (12) may be actuated through a single layer of tissue (90) or more than two layers (92, 94) of tissue. It should also be understood that the formation and positioning of staples (47) adjacent to the cut line produced by cutting edge (48) may substantially seal the tissue at the cut line, thereby reducing or preventing bleeding and/or leaking of other bodily fluids at the cut line. Various suitable settings and procedures in which instrument (10) may be used will be apparent to those of ordinary skill in the art in view of the teachings herein.

It should be understood that instrument (10) may be configured and operable in accordance with any of the teachings of U.S. Pat. No. 4,805,823; U.S. Pat. No. 5,415,334; U.S. Pat. No. 5,465,895; U.S. Pat. No. 5,597,107; U.S. Pat. No. 5,632,432; U.S. Pat. No. 5,673,840; U.S. Pat. No. 5,704,534; U.S. Pat. No. 5,814,055; U.S. Pat. No. 6,978,921; U.S. Pat. No. 7,000,818; U.S. Pat. No. 7,143,923; U.S. Pat. No. 7,303,108; U.S. Pat. No. 7,367,485; U.S. Pat. No. 7,380,695; U.S. Pat. No. 7,380,696; U.S. Pat. No. 7,404,508; U.S. Pat. No. 7,434,715; and/or U.S. Pat. No. 7,721,930.

Additional exemplary modifications that may be provided for instrument (10) will be described in greater detail below. Various suitable ways in which the below teachings may be incorporated into instrument (10) will be apparent to those of ordinary skill in the art. Similarly, various suitable ways in which the below teachings may be combined with various teachings of the patents cited herein will be apparent to those of ordinary skill in the art. It should also be understood that the below teachings are not limited to instrument (10) or devices taught in the patents cited herein. The below teachings may be readily applied to various other kinds of instruments, including instruments that would not be classified as surgical staplers. Various other suitable devices and settings in which the below teachings may be applied will be apparent to those of ordinary skill in the art in view of the teachings herein.

### II. Exemplary Cartridge with Film

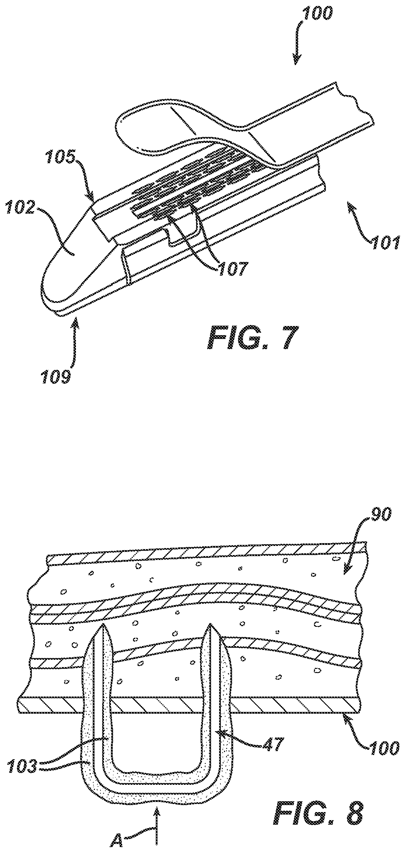

FIG. 7 shows another exemplary removable cartridge (101) that may be inserted into lower jaw (16) of end effector (12) shown in FIG. 2. Other than as set forth below, cartridge (101) of this example is similar to cartridge (37) describe above. A buttress (100) is disposed above the top surface (102) of the upper deck (105) of cartridge (101). Buttress (100) comprises a film that delivers a hemostatic agent to tissue (90), as described below. Alternatively, buttress (100) may have any other suitable properties.

Staple pockets (107) of cartridge (101) are similar to staple apertures (51) described above and are configured to receive a foam, paste, or gel material after cartridge (101) is disposed in lower jaw (16). The material contained in pockets (107) is configured to keep staples (47) in place in pockets (107) and/or to seal the medicated, biocompatible material within body (109) of cartridge (101). Pockets (107) may include various materials, such as glue, fabric, and materials that would be apparent to those of ordinary skill in the art in view of the teachings herein. A film such as buttress (100) comprising a hemostatic agent is disposed on top surface (102) to cover gel-filled pockets (107) and is heated to secure buttress (100) and the underlying medicated material into place. In some versions, only the outer edges of buttress (100) are heated to secure buttress (100) to cartridge (101). Any suitable devices may be used to provide such heating, including but not limited to an anvil (e.g., a custom thermoform die on a light press, etc.). While buttress (100) is shown as disposed over cartridge (101), buttress (100) may additionally or alternatively be disposed on an undersurface of anvil (18) that faces cartridge (101).

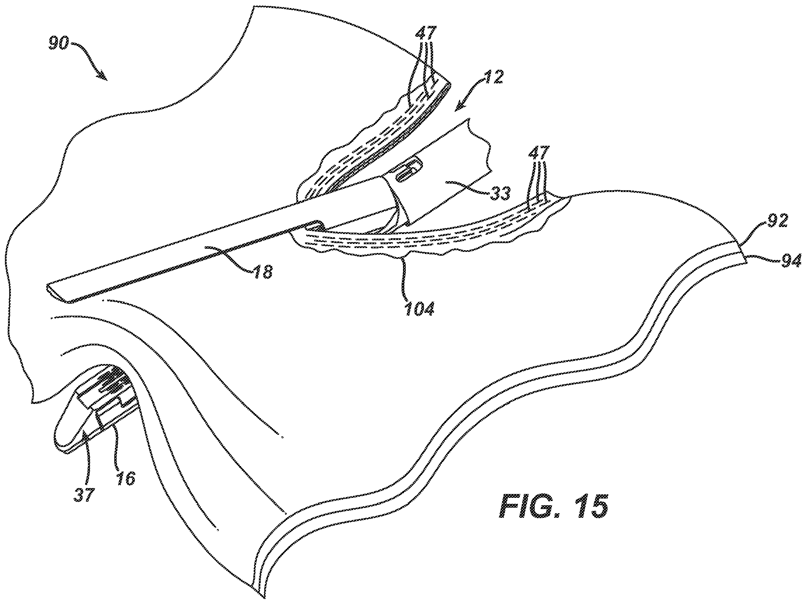

FIG. 8 shows a staple (47) being driven in a manner described above, in the direction of arrow (A), toward anvil (18) through buttress (100) and into layers of tissue (90). In some versions, staples (47) and drivers (43) may also be coated with a hemostatic agent or other adjunct material (described below), which may act as an activating agent to react with buttress (100). For example, staples (47) and drivers (43) may be coated with one of fibrin or thrombin, while buttress (100) may comprise the other of fibrin or thrombin. As coated staples (47) are driven in the direction of arrow (A) through gel-filled pockets (107) to puncture buttress (100), staples (47) release a tissue repair composition material from both staples (47) and buttress (100) onto and into tissue (90). For example, FIG. 8 shows gel (103) from gel-filled pockets (107) coated on staple (47). Additionally, when end effector (12) including cartridge (101) and buttress (100) is used, firing bar (14) is fired into tissue (90) while slicing through buttress (100) to release a tissue repair composition material from buttress (100) onto tissue (90). The tissue repair composition material may be released as the tissue repair composition (104) shown in FIG. 15 when end effector (12) including cartridge (101) staples tissue (90) with staples (47). In addition or in the alternative, material from buttress (100) may provide reinforcement to the integrity of the mechanical attachment of layers (92, 94) of tissue (90) by staples (47). Surgical staple (47) may comprise a material selected from iron, nickel titanium alloy, stainless steel, and/or titanium. Of course, any other suitable materials may be used.

The material for buttress (100) as well as the material disposed in pockets (107) and coated on staples (47) may comprise, for example, adjunct or hemostatic agents such as fibrin or thrombin that assist to coagulate blood and reduce the amount of bleeding at the surgical site. The hemostatic abilities of such adjuncts may also contribute to the use of such adjuncts as adhesives and sealants. The agents may assist to coagulate blood at a surgical site which allows tissue surrounding such blood to stick together and may prevent leaks along the stapled tissue site, for example.

Such adjuncts or reagents may further include but are not limited to medical fluid or buttress components such as platelet poor plasma (PPP), platelet rich plasma (PRP), starch, chitosan, alginate, fibrin, polysaccharide, cellulose, collagen, bovine collagen, gelatin-resorcin-formalin adhesive, oxidized cellulose, mussel-based adhesive, poly (amino acid), agarose, amylose, hyaluronan, polyhydroxybutyrate (PHB), hyaluronic acid, poly(vinyl pyrrolidone) (PVP), poly(vinyl alcohol) (PVA), polylactide (PLA), polyglycolide (PGA), polycaprolactone (PCL), and their copolymers, VICRYL® (Ethicon, Inc., Somerville, N.J.), MONOCRYL material, PANACRYL (Ethicon, Inc., Somerville, N.J.), and/or any other material suitable to be mixed with biological material and introduced to a wound or defect site, including combinations of materials. For example, buttress (100) may comprise a material selected from the following materials: epsilon-caprolactone glycolide, bovine pericardium, polylactic acid, polyglycolic acid, polyglactin, polydioxanone, polyglyconate, whey protein, cellulose gum, starch, gelatin, silk, nylon, polypropylene, braided polyester, polybutester, polyethylene, and/or polyetheretherketones. Other suitable compounds, materials, substances, etc., that may be used in a medical fluid or buttress will be apparent to those of ordinary skill in the art in view of the teachings herein.

In some versions, a medical fluid may be suspended in a biocompatible carrier to form the material of buttress (100). Suitable carriers may include, for example, a physiological buffer solution, a flowable gel solution, saline, and water. In the case of gel solutions, the tissue repair composition may be in a flowable gel form prior to delivery at the target site, or may form a gel and remain in place after delivery at the target site. Flowable gel solutions may comprise one or more gelling materials with or without added water, saline, or a physiological buffer solution. Suitable gelling materials include biological and synthetic materials. Exemplary gelling materials include proteins, polysaccharides, polynucleotides, and other materials such as alginate, cross-linked alginate, poly(N-isopropylacrylamide), poly(oxyalkylene), copolymers of poly(ethylene oxide)-poly(propylene oxide), poly(vinyl alcohol), polyacrylate, or monostearoyl glycerol co-Succinate/polyethylene glycol (MGSA/PEG) copolymers, and combinations of any of the foregoing.

Buttress (100) may comprise a fibrous pad, a foam, a matrix, a mesh, or another structure, in accordance with the teachings of, by way of example, U.S. Patent App. Pub. No. 2009/0120994, entitled "Surgical Fastening Device with Initiator Impregnation of a Matrix or Buttress to Improve Adhesive Application", published May 14, 2009. The material may comprise, for example, a biocompatible material that is a buttress, a matrix having a plurality of openings therein, an open cell or closed cell foam, and/or a fabric pad. The material may include porosities that induce a wicking feature to drawing adhesive into the material and ensure the openings remain clear of the adhesive, allowing tissue growth through the openings after application to tissue.

Additionally or alternatively, buttress (100) may be comprised of an adhesive such as, but not limited to, polymerizable and/or cross-linkable materials such as a cyanoacrylate adhesive. The adhesive, for example, may be a monomeric (including prepolymeric) adhesive composition, a polymeric adhesive composition, or any other compound that can adhere to tissue. In embodiments, the monomer may be a 1,1-disubstituted ethylene monomer, e.g., an alpha-cyanoacrylate. When cross linked or polymerized, the cyanoacrylate can change from a liquid to a solid. Polymerized adhesives for example, can be formulated to be flexible to rigid and could be spongy. If desired, the adhesive can be a single part or dual part adhesive, and/or can contain additives such as alternate compounds. Polymerization of the adhesive can occur from, but is not limited to, exposure to moisture, heat, and/or adhesion initiators such as those described in U.S. Patent App. Pub. No. 2009/0120994. Other suitable materials and compositions that may be used to form buttress (100) will be apparent to those of ordinary skill in the art in view of the teachings herein.

### III. Exemplary Applicator

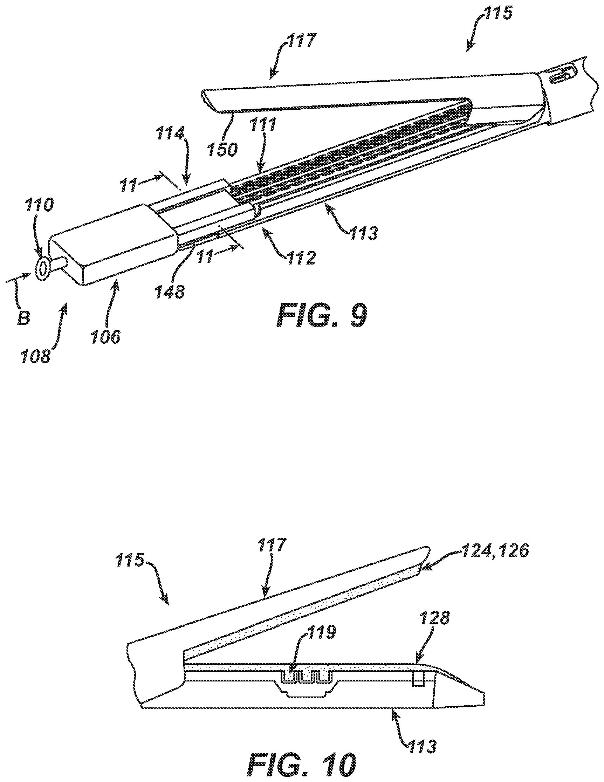

FIGS. 9-12 are associated with use of exemplary disposable applicator (106). FIG. 9 shows applicator (106) being positioned onto the upper deck (111) of a cartridge (113) in a version of end effector (115), which has components similar to end effector (12) described above. For example, the anvil (117) of end effector (115) in this example is similar to anvil (18) of end effector (12). Applicator (106) includes a first end (108) with a handle (110) to assist with urging applicator (106) onto cartridge (113). Handle (110) also assists with removing applicator (106) from cartridge (113) after material from applicator (106) is applied to anvil (117) and cartridge (113), as described below. A second end (112) of applicator (106) includes applicator portion (114) including material for deposit onto anvil (117) and cartridge (113).

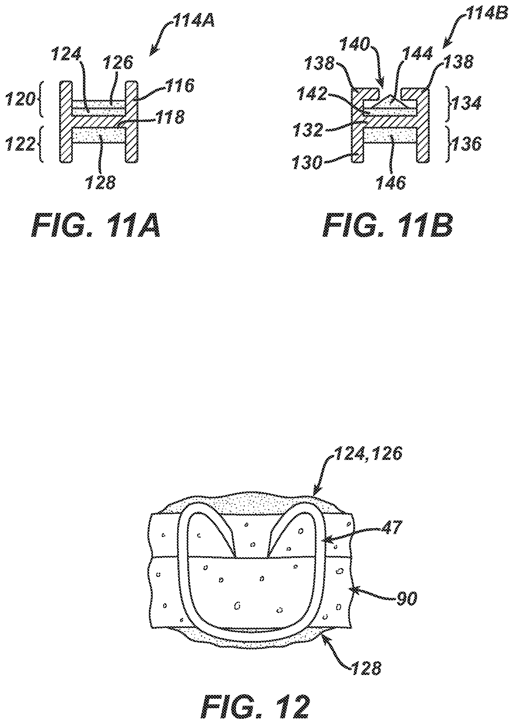

FIGS. 11A and 11B show alternative versions of cross-sections of applicator portion (114). FIG. 11A shows a version in which applicator portion (114A) presents an H-shaped cross-section including sidewalls (116) and central wall (118) disposed between sidewalls (116) to form upper portion (120) and lower portion (122). Upper portion (120) includes two layers (124, 126) of material. The first layer (124) of material may comprise a material such as fabric disposed atop wall (118). The fabric may comprise, for example, a woven Oxidized Regenerated Cellulose (ORC) that forms a buttress protecting an adjunct gel. Of course, any other suitable material (e.g., woven or nonwoven fabric, mesh, or textile, etc.) may be used. The second layer (126) of material may comprise a type of paste or gum (e.g., adhesive such as cyanoacrylate, etc.), or any other suitable material, disposed atop the first layer (124). Lower portion (122) includes a single layer (128) of material such as hemostatic adjunct gel or any other suitable material. Either side of wall (118) may include any number of layers of material.

FIG. 11B shows a version in which applicator portion (114B) presents a cross-section including sidewalls (130) and central wall (132) disposed between sidewalls (130) to form upper portion (134) and lower portion (136). Upper portion (134) includes two mutually facing arms (138), each arm (138) extending inwardly from a respective sidewall (130). Arms (138), sidewalls (130), and central wall (132) also form an inverted T-shaped channel (140) within upper portion (134). Arms (138) assist with retaining material within T-shaped channel (140), which retains two layers (142, 144) of material. The first layer (142) of material may comprise a material such as fabric disposed atop central wall (132). Similar to portion (114A), the fabric may comprise, for example, a woven ORC forming a buttress to protect an adjunct gel. The second layer (144) of material may comprise a type of gum or paste, or any other suitable material, disposed atop the first layer (142). Lower portion (136) includes a single layer (146) of material such as hemostatic adjuct gel, or any other suitable material. Either side of wall (132) may include any number of layers of material.

Referring back to FIG. 9, regardless of which applicator portion (114A, 114B) is used, applicator (106) is directed onto cartridge (113) to apply gel (128) onto deck (111) of cartridge (113). In application, sidewalls (116, 130) of applicator (106) are sized for slidable receipt in the direction of arrow (B) onto sidewalls (148) of cartridge (113). Applicator (106) locates and aligns easily on cartridge (113). When applicator portion (114A) of applicator (106) is fully received on deck (111) of cartridge (113), a user may direct anvil (117) towards cartridge (113) as described above. For example, a user may pivotally draw closure trigger (26) towards handle portion (20) to longitudinally translate closure sleeve (32) towards anvil (117) in response to the pivotal drawing motion, and closure sleeve (32) provides for closing of anvil (117). When anvil (117) presses against applicator portion (114A), undersurface (150) of anvil (117) will press against gum (126), which will act as an adhesive to attach to undersurface (150). Further, the pressure of anvil (117) against applicator portion (114A) and deck (111) will urge gel (128) into pockets (119) of deck (111). Pockets (119) filled with gel (128) are shown in FIG. 10, for example. The pressure of anvil (117) against applicator portion (114A) and deck (111) will also deposit gel (128) substantially along a top surface of deck (111).

Additionally, when anvil (117) is directed away from deck (111) of cartridge (113) by releasing closure trigger (26), which longitudinally translates closure sleeve (32) away from anvil (117), gum (126) will pull fabric (124) out of upper portion (120) of applicator portion (114A) and hold it to undersurface (150) of anvil (117). A user may then use handle (110) to pull applicator (106) away from cartridge (113) and may then dispose of applicator (106). When applicator (106) is pulled off of cartridge (113) in a direction substantially opposite to arrow (B), and where cartridge (113) is outside a patient, material (124, 126, 128) has been applied onto end effector (115), which is ready for use in a patient. FIG. 10 shows end effector (115) after applicator (106) has been used to apply material (124, 126, 128) onto end effector (12).

When end effector (115) is used in a manner similar to that described above for end effector (12), firing bar (14) will slice through gel (128) and release biocompatible gel (128) onto sliced layers (92, 94) of tissue (90). Concurrently, firing bar (14) will slice through buttress fabric (124) and adhesive gum (126) to release an adjunct gel contained in fabric (124) and adhesive from gum (126) onto sliced and severed tissue (90). Additionally, staples (47) will be driven through material (124, 126, 128) and into tissue (90) such that staples (47) capture material (124, 126, 128) and deposit it onto captured layers (92, 94) of tissue (90) as shown in FIG. 12. A new cartridge (113) may then be reloaded and a new applicator (106) may be used to apply a new layer of material (124, 126, 128) onto end effector (115) after each firing of cartridge (113) via end effector (115).

Gum (126) may comprise an adhesive such as, but not limited to, polymerizable and/or cross-linkable materials such as a cyanoacrylate adhesive. The adhesive, for example, may be a monomeric (including prepolymeric) adhesive composition, a polymeric adhesive composition, or any other compound that can adhere to tissue. In embodiments, the monomer may be a 1,1-disubstituted ethylene monomer, e.g., an alpha-cyanoacrylate. When cross linked or polymerized, the cyanoacrylate can change from a liquid to a solid. Polymerized adhesives for example, can be formulated to be flexible to rigid and could be spongy. If desired, the adhesive can be a single part or dual part adhesive, and/or can contain additives such as alternate compounds. Polymerization of the adhesive can occur from, but is not limited to, exposure to moisture, heat, and/or adhesion initiators such as those described in U.S. Patent App. Pub. No. 2009/0120994. Other suitable materials and compositions that may be used to form gum (124) will be apparent to those of ordinary skill in the art in view of the teachings herein.

### III. Exemplary Capillary Action

FIG. 13 shows a view of exemplary cartridge (121), which has components similar to cartridge (37) described above. Buttress (152) is disposed on upper deck (153) of cartridge (121) and may comprise a film, bladder, reservoir, or other suitable material/strucutre. Referring to FIG. 13, buttress (152) houses antibiomaterial (154) which may comprise, for example, a liquid sealant comprised of a hemostatic material as described above for delivery via capillary force to tissue (90) punctured by staple (47) of cartridge (121). For example, when staple (47) driven by wedge sled (41) and driver (43) punches through buttress (152), a gap between the legs of staple (47) and tissue (90) may provide a path for a capillary force that may pull antibiomaterial (154) along the legs of staple (47) and onto tissue (90).

### IV. Exemplary Glue Filled Buttress Bag

FIG. 14 shows an exemplary cartridge (123) that has components substantially similar to cartridge (37) described above. For example, cartridge (123) includes a metal deck (127), with a longitudinal slot (125) running through a central portion of deck (127). Staples (47) are housed in a body or housing (129) of cartridge (123) under apertures (not shown) formed in deck (127) on either side of slot (125). Cartridge (123) differs from cartridge (37) as set forth below.

Cartridge (123) includes an envelope (156) disposed above staples (47) in housing (129) and below deck (127). Envelope (156) is retained in a pocket (158) formed by internal walls of housing (129) below deck (127) and above housed staples (47). Envelope (156) may comprise a glue filled buttress material comprised of an adhesive and buttress material as respectively described above for gum (126) of cartridge (113) and buttress (100) of cartridge (101). Of course, any other suitable materials and configurations may be used as apparent to one of ordinary skill in the art in view of the teachings herein.

Firing bar (14) and staples (47) may both be coated with a material, such as an adhesive or other liquid biocompatible material, to assist with application of the material from envelope (156) onto tissue (90), which firing bar (14) and staples (47) sever and staple in a manner described above for cartridge (37). Additionally, the glue in envelope (156) may be substituted with a fibrin or thrombin biologic agent. For example, firing bar (14) and staples (47) may be coated with a material such as thrombin to react with the material retained in envelope (156), which may be fibrin for example, when firing bar (14) and staples (47) puncture envelope (156). The alternative application of fibrin and thrombin is possible, such that firing bar (14) and staples (47) are coated with fibrin and envelope (156) comprises thrombin. Indeed, a wide variety of synthetic and biologic agents may be used. Such material may be applied to cartridge (123) at a manufacturing site or sold separately and applied at a later stage. Alternatively, cartridge (123) may include two compartments to contain two separate glues or any other suitable biocompatible materials.

It should be understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures.

Versions of described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the systems described herein may be accomplished by appropriate modifications by one of ordinary skill in the art without departing from the scope of the present disclosure. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument apparatus (10) comprising:
(a) a handle portion (20);
(b) a shaft housing (23) a firing bar (14);
(c) an end effector (115) comprising an anvil (117), a lower jaw (16), and a stapling and severing assembly responsive to a longitudinal closing motion produced by the handle portion and the shaft;
(d) a removable cartridge (101), wherein the lower jaw is configured to receive the cartridge, the cartridge comprising:
(i) a housing (129),
(ii) a plurality of staples (47) disposed in the housing,
(iii) a deck (153) disposed over the plurality of staples, the deck defining apertures, wherein each aperture is substantially disposed over a respective staple,
(iv) at least a first biocompatible material disposed in the cartridge, and
(v) at least a second biocompatible material (154) disposed on the deck;
**characterised in that** the surgical instrument apparatus comprises:
(e) a pocket (158) and an envelope (156), wherein the pocket is defined in the cartridge,
wherein the pocket is disposed below the deck and above the plurality of staples, wherein the pocket is configured to receive the envelope, and wherein the envelope comprises the first biocompatible material.

2. The apparatus of claim 1, wherein the envelope (156) comprises a buttress (152), and wherein the first biocompatible material comprises an adhesive.

3. The apparatus of claim 2, wherein the buttress (152) is configured to contain the adhesive.

4. The apparatus of claim 3, wherein the adhesive is comprised of a monomeric adhesive composition or a polymeric adhesive composition.

5. The apparatus of claim 1, wherein the envelope (156) comprises a buttress, and wherein the biocompatible material comprises one of fibrin or thrombin.

6. The apparatus of claim 5, wherein each staple (47) is coated with the other of fibrin or thrombin.

## Patentansprüche

1. Chirurgische Instrumentenvorrichtung (10), umfassend:
(a) einen Griffabschnit (20);
(b) ein Schaftgehäuse (23), einen Abfeuerstab (14) ;
(c) einen Endeffektor (115), umfassend einen Amboss (117), eine untere Backe (16) und eine Klammer- und Durchschneideanordnung, die auf eine vom Griffabschnitt und vom Schaft erzeugte, längs gerichtete Schließbewegung anspricht;
(d) eine entfernbare Kassette (101), wobei die untere Backe zur Aufnahme der Kassette ausgelegt ist, wobei die Kassette das Folgende umfasst:
(i) ein Gehäuse (129),
(ii) eine Vielzahl von Klammern (47), die im Gehäuse angeordnet sind,
(iii) ein Deck (153), das über der Vielzahl von Klammern angeordnet ist, wobei das Deck Öffnungen definiert, wobei jede Öffnung im Wesentlichen über einer jeweiligen Klammer angeordnet ist,
(iv) mindestens ein erstes biokompatibles Material, das in der Kassette angeordnet ist, und
(v) mindestens ein zweites biokompatibles Material (154), das auf dem Deck angeordnet ist;
**dadurch gekennzeichnet, dass** die chirurgische Instrumentenvorrichtung das Folgende umfasst:
(e) eine Tasche (158) und eine Hülle (156), wobei die Tasche in der Kassette definiert ist,
wobei die Tasche unter dem Deck und über der Vielzahl von Klammern angeordnet ist, wobei die Tasche zur Aufnahme der Hülle ausgelegt ist und
wobei die Hülle das erste biokompatible Material umfasst.

2. Vorrichtung nach Anspruch 1, wobei die Hülle (156) eine Versteifung (152) umfasst, und wobei das erste biokompatible Material einen Klebstoff umfasst.

3. Vorrichtung nach Anspruch 2, wobei die Versteifung (152) dazu ausgelegt ist, den Klebstoff zu enthalten.

4. Vorrichtung nach Anspruch 3, wobei der Klebstoff aus einer monomeren Klebstoffzusammensetzung oder einer polymeren Klebstoffzusammensetzung besteht.

5. Vorrichtung nach Anspruch 1, wobei die Hülle (156) eine Versteifung umfasst und wobei das biokompatible Material Fibrin oder Thrombin umfasst.

6. Vorrichtung nach Anspruch 5, wobei jede Klammer (47) mit dem jeweils anderen von Fibrin oder Thrombin beschichtet ist.

## Revendications

1. Appareil d'instrumentation chirurgicale (10) comprenant :
(a) une partie manche (20) ;
(b) une tige (23) recevant une barre de déclenchement (14) ;
(c) un effecteur terminal (115) comprenant une enclume (117), une mâchoire inférieure (16) et un ensemble d'agrafage et de séparation sensible à un mouvement de fermeture longitudinale produit par la partie manche et la tige ;
(d) une cartouche amovible (101), la mâchoire inférieure étant configurée pour recevoir la cartouche, la cartouche comprenant :
(i) un boîtier (129),
(ii) une pluralité d'agrafes (47) disposées dans le boîtier,
(iii) un plateau (153) disposé sur la pluralité d'agrafes, le plateau définissant des ouvertures,
chaque ouverture étant sensiblement disposée sur une agrafe respective,
(iv) au moins un premier matériau biocompatible disposé dans la cartouche, et
(v) au moins un second matériau biocompatible (154) disposé sur le plateau ;
**caractérisé en ce que** l'instrument chirurgical comprend :
(e) une poche (158) et une enveloppe (156), la poche étant définie dans la cartouche,
la poche étant disposée sous le plateau et au-dessus de la pluralité d'agrafes, la poche étant configurée pour recevoir l'enveloppe, et l'enveloppe comprenant le premier matériau biocompatible.

2. Appareil selon la revendication 1, l'enveloppe (156) comprenant un contrefort (152), et le premier matériau biocompatible comprenant un adhésif.

3. Appareil selon la revendication 2, le contrefort (152) étant configuré pour contenir l'adhésif.

4. Appareil selon la revendication 3, l'adhésif étant constitué d'une composition adhésive monomère ou d'une composition adhésive polymère.

5. Appareil selon la revendication 1, l'enveloppe (156) comprenant un contrefort, et le matériau biocompatible comprenant l'une de la fibrine ou de la thrombine.

6. Appareil selon la revendication 5, chaque agrafe (47) étant revêtue de l'autre de la fibrine ou de la thrombine.
